# EUROPÄISCHE PATENTSCHRIFT

(11) **EP 2 471 558 B1**
(45) Veröffentlichungstag und Bekanntmachung des Hinweises auf die Patenterteilung: **12.07.2017**
(21) Anmeldenummer: 12002197.7
(22) Anmeldetag: 30.07.2008
(51) Int. Cl.: A61L 2/07, B65D 71/50, B65B 55/02

(54) **Anlage zur Sterilisation von Gegenständen, vorzugsweise mit Hilfe eines Dampf-Luft-Gemisches**
Facility for the sterilisation of articles, preferably with a mixture of air and steam
Installation pour la stérilisation d'objets, de préférence avec un mélange de vapeur et d'air

(30) Priorität: 03.08.2007 DE 102007036734
(43) Veröffentlichungstag der Anmeldung: 04.07.2012
(62) Teilanmeldung aus: 08785204.2
(73) Patentinhaber: Klosterfrau Berlin Gmbh, 12277 Berlin (DE)
(72) Erfinder: Venten, Ernst, verstorben (DE)
(74) Vertreter: Von Rohr Patentanwälte Partnerschaft mbB

(56) Entgegenhaltungen:
- EP-A2- 0 745 536
- WO-A-03/074093
- WO-A1-02/20066
- WO-A1-2007/056591
- CH-A- 223 434

## Beschreibung

Die vorliegende Erfindung betrifft eine Anlage zur Sterilisation von Gegenständen, vorzugsweise mit Hilfe eines Dampf-Luft-Gemische.

Im medizinischen Bereich und darüber hinaus in vielen anderen, auch industriellen, Bereichen, in denen Sterilität ein Qualitätsmerkmal ist, werden eine Vielzahl von Sterilisationsverfahren angewendet. Typischerweise werden dabei Gegenstände in Sterilisationskammern eingebracht. Bei kostengünstigen Massenproduktionsverfahren ist es jedoch erforderlich, nicht nur effizient sondern auch kostengünstig eine Vielzahl von Gegenständen, insbesondere auch gleichartigen Gegenständen, zu sterilisieren.

Bei einem bekannten Sterilisationsverfahren (EP 0 703 793 B1) können Gegenstände, die in einer Faltschachtel verkauft werden sollen, zusammen mit dieser Faltschachtel sterilisiert werden. Die Gruppierung von Gegenständen in einer Faltschachtel ermöglicht die effiziente Handhabung einer großen Anzahl zu sterilisierender Gegenstände. Allerdings werden dafür gewisse Kompromisse eingegangen. Insbesondere wenn mit einem Dampf-Luft-Gemisch sterilisiert werden soll, ist die effiziente Heranführung des Dampf-Luft-Gemisches an die Gegenstände und die effiziente Umwälzung des Dampf-Luft-Gemisches wichtig. Im Übrigen ist in aller Regel eine Sterilisation der äußern Umverpackung, also etwa der Faltschachtel, nicht erforderlich, da bei späteren Handhabungsschritten diese Umverpackung ohnehin nicht steril gehalten werden kann.

in einem weiteren Aspekt, der gerade für die Bereitstellung steriler medizinischer Gegenstände eine Rolle spielt, ist es wichtig, dass das Erscheinungsbild der äußeren Verpackung durch das Sterilisationsverfahren nicht leidet. Wenn aber beispielsweise Faltschachteln einer typischen Dampf-Luft-Sterilisation ausgesetzt sind, weisen nicht selten einzelne Faltschachteln Verfärbungen oder Wasserflecken auf. Sofern die Faltschachteln auf Gestellten oder dergleichen angeordnet sind, entstehen während eines Dampf-Luft-Sterilisationsprozesses auch leicht Druckstellen. Häufig sind solche Druckstellen, wie auch die Wasserflecken, dauerhaft sichtbar.

Wünschenswert wäre es also, den offenbarten Prozess seiner Qualität nach zu verbessern, ohne aber die Kasteneffzienz leiden zu lassen. Idealerweise wird eine Qualitätsverbesserung bei zugleich höherer Wirtschaftlichkeit erreicht- Insbesondere wäre es auch wünschenswert, einen Prozess zu gestalten, der mit handelsüblichen Einrichtungen und Apparaten auskommt, und der möglicherweise auf einer solchen Sterilisationsanlage ausgeführt werden kann, wie sie bislang für die Sterilisation, mit Faltschachtein eingesetzt wird.

Die benannte Anlage zur Sterilisation von Gegenständen, von der die Erfindung ausgeht (CH-A-223-434), ist eine halbautomatisch arbeitende Anlage. Die betroffenen Gegenstände, die sterilisiert werden sollen, werden hier als Bündel zusammengestellt und in einen topfförmige Sterilisationsraum eingesetzt. Nach dem Sterilisieren werden sie auf nicht weiter beschriebene Weise aus dem Sterilisationsraum herausgenommen. Das muss offensichtlich mit einer sterilen Handhabungsweise geschehen.

Der Lehre liegt das Problem zugrunde, eine verbesserte Anlage zur Sterilisation von Gegenständen vorzuschlagen, die weitgehend automatisch arbeitet, so dass damit ein zeit- und kosteneffizientes Sterilisieren erreichbar ist.

Die zuvor aufgezeigte Problemstellung ist bei einer Anlage mit den Merkmalen von Anspruch 1 gelöst. Bevorzugte Ausgestaltungen und Weiterbildungen sind Gegenstand der Unteransprüche.

Für das von der Anlage auszuführende Verfahren wird ein Umhüllungsmittel eingesetzt.

Dieses Umhüllungsmittel ist so auszulegen, dass es eine Gruppe von Gegenständen teilweise, aber nicht vollständig umhüllt. In Vorbereitung ihrer Sterilisation, können die Gegenstände in günstiger Weise in Gruppen angeordnet werden. Je eine Gruppe von Gegenständen wird dann mit dem Umhüllungsmittel umhüllt. Da diese Umhüllung die Gegenstände nur teilweise, aber nicht vollständig bedeckt, wird die Sterilisation begünstigt, insbesondere kann an den unverhüllten Seiten ein Dampf-Luft-Gemisch eintreten und auch austreten. Dabei kann der Eintritt und der Austritt an gegenüberliegenden Seiten der Gruppe von Gegenständen sein. Die Umhüllung ihrerseits stellt sicher, dass die Anordnung der Gruppe von Gegenständen erhalten bleibt. Diese Anordnung kann bezogen auf die Sterilisation und/oder auf die spätere Verpackung optimiert sein.

Das von der Anlage auszuführende Verfahren eignet sich besonders gut auch zur Sterilisation eher länglicher Gegenstände. Diese Gegenstände können dann entlang ihrer längsten Länge parallel angeordnet werden, und zwar so, dass zwischen ihnen Durchströmkanäle für ein Gas-Luft-Gemisch verbleiben. Die Umhüllung wird dann so angebracht, dass ein Bündel von Gegenständen entsteht und das Umhüllungsmaterial dem Rand einer Fläche folgt (oder den Rändern einer Vielzahl paralleler Flächen), die im Wesentlichen senkrecht zu den Längsachsen der Gegenstände ist. Dies ermöglicht eine gute mechanische Stabilisierung der Gruppe und erhält zugleich günstige Durchströmkanäle für den Sterilisationsprozess.

Insbesondere ist das von der Anlage auszuführende Verfahren auch für unregelmäßig geformte Gegenstände geeignet, die über konkav-konvexe Konturen verfügen, welche sich aber so anordnen lassen, dass eine (konvexe)Ausbuchtung des einen Gegenstandes in eine (konkave) Einbuchtung des anderen Gegenstandes eingreift. Gerade solche Gegenstände lassen sich raumsparend in Gruppen anordnen und eventuell in mindestens einem Querschnitt oder vollständig formschlüssig anordnen, was für den Sterilisationsprozess erhebliche Kostenvorteile bringt. Durch die konkav-konvexe Form der Gegenstände ergeben sich jedoch gebogene Durchströmkanäle. Wenn die Einlass- oder Auslassöffnungen solche Durchströmkanäle durch Verpackungsmaterial oder dergleichen blockiert werden, ist eine effiziente Sterilisierung erschwert. Eine Umhüllung von Seiten, an denen solche Durchströnnkanäle nur in kleiner Zahl, oder mit kleinem Querschnitt oder gar nicht vorhanden sind, ist jedoch weniger problematisch.

Insbesondere kann die Umhüllung auch dazu dienen, eine erfolgreiche Sterilisation anzuzeigen. Dies bietet sich insbesondere deshalb an, weil die Umhüllung annähernd identischen Sterilisationsbedingungen ausgesetzt ist wie die umhüllte Gruppe von Gegenständen. Zu diesem Zweck kann ein Indikator an der Umhüllung angebracht werden. Ein geeigneter Indikator ist ein Farbindikator, der bei einem definierten "F0-Wert" umschlägt. In einer bevorzugten Ausführung ist der Farbindikator automatisch lesbar.

Insbesondere kann die Umhüllung auch als Originalitätsverschluss dienen. Ein solcher Verschluss soll einem Käufer oder Benutzer der Gegenstände anzeigen, dass sie sich noch im Originalzustand befinden, das heißt in dem Zustand wie hergestellt, insbesondere auch wie sterilisiert.

Insbesondere kann die Umhüllung auch dazu dienen, eine Transportmöglichkeit für die manuelle oder maschinelle Handhabung einer Gruppe von Gegenständen anzubieten. Als Transportmöglichkeit kommt das Greifen der Umhüllung an beliebiger Stelle in Frage, vorzugsweise ist die Umhüllung mit einer Greifvorrichtung, z. B. einer Griffschlaufe versehen.

Die erfindungsgemäße Anlage zur Sterilisation von Gegenständen, mit der die zuvor nochmals erläuterten Verfahrensschritte komplett oder überwiegend umgesetzt werde können, ist zunächst gekennzeichnet durch folgende Bestandteile:
a) einen Sterilisationsraum,
b) eine automatische Vorrichtung zur Anordnung der Gegenstände in Gruppen, wobei die Gegenstände in mindestens einer Gruppe so angeordnet werden, dass ihre Hauptachsen L parallel zueinander ausgerichtet sind und wobei die Gegenstände der Gruppe mit einer Umhüllung versehen wird, so dass ein Bündel von Gegenständen entsteht und das Umhüllungsmaterial dem Rand einer Fläche folgt oder den Rändern einer Vielzahl paralleler Flächen folgt, die im Wesentlichen senkrecht zu den Längsachsen der Gegenstände ist oder sind
c) eine automatische Vorrichtung, um je eine Gruppe von Gegenständen mit einer die Gruppe teilweise, aber nicht vollständig bedeckenden Umhüllung zu versehen,
d) Vorrichtungen, um mindestens eine Gruppe von Gegenständen einschließlich der die Gruppe teilweise, aber nicht vollständig bedeckenden Umhüllung in den Sterilisationsraum einzubringen,
e) Vorrichtungen zur Entnahme der Gegenstände der Gruppe als Bündel einschließlich ihrer Umhüllung aus dem Sterilisationsraum,
f) automatische Vorrichtungen, um je eine Gruppe von Gegenständen einschließlich ihrer Umhüllung in ein Verpackungsmittel einzubringen, das zur vollständigen Umhüllung der Gruppe von Gegenständen geeignet ist.

Insbesondere empfiehlt es sich, dass die Vorrichtungen gemäß Merkmal d) einen Sterilisationskorb umfassen.

Diese und noch weitere Merkmale der Erfindung gehen aus den Ansprüchen hervor, aber auch aus der nachfolgenden Beschreibung und den zugehörigen Zeichnungen. Zu beachten ist, dass Merkmale, die in einem bestimmten Zusammenhang offenbart werden, auch als kombinierbar mit anderen Merkmalen, die im Einzelfall nur in anderen Zusammenhängen offenbart werden, aufzufassen sind. Die Erfindung wird anhand folgender Zeichnungen näher erläutert.
- Fig. 1: schematischer Ablauf des mit der erfindungsgemäßen Anlage praktizierten Verfahrens.
- Fig. 2: zeigt Gegenstände, die zu einer Gruppe angeordnet sind.
- Fig. 3: zeigt einen Längsschnitt entlang der Linie III-III aus der Fig. 2.
- Fig. 4: zeigt eine Gruppe von Gegenständen zusammen mit einer Umhüllung.
- Fig. 5: zeigt einen Längsschnitt entlang der Linie V-V durch die Gruppe von Gegenständen aus Fig. 4.
- Fig. 5A: zeigt einen Längsschnitt nach Art der Fig. 5, jedoch für eine Ausführungsform, bei der die Umhüllung zusätzlich eine Transportmöglichkeit in Form einer Griffschlaufe aufweist.
- Fig. 5B: zeigt einen Längsschnitt nach Art der Fig. 5, jedoch für eine Ausführungsform, bei der die Umhüllung als Stülpboden ausgeführt ist.
- Fig. 6: zeigt die gleiche Gruppe von Gegenständen wie Fig. 2 und Fig. 4 in der Draufsicht und nach dem Einbringen in ein Verpackungsmittel.
- Fig. 7: zeigt eine Ansicht englang der Linie VII-VII aus Fig. 6.

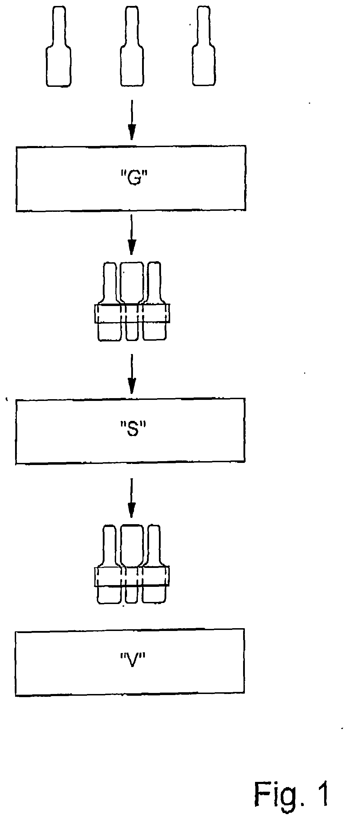

In der Fig. 1 sind die Grundzüge des Mittels der erfindungsgemäßen Anlage umsetzbaren Verfahrens schematisch dargestellt. Dabei steht "G" für das Gruppieren von Gegenständen, "S" steht für das Sterilisieren von Gegenständen und "V" steht für das Verpacken von einer Gruppe von Gegenständen. Gegenstände (10) werden zunächst automatisch zu geeigneten Gruppen (20) zusammengefasst. Die so gebildete mindestens eine Gruppe (20) von Gegenständen (10) werden im nächsten Schritt mit einer Umhüllung (30) versehen. Diese Umhüllung (30) bedeckt die Gruppe (20) von Gegenständen (10) teilweise, jedoch nicht vollständig. Die Gruppe (20) von Gegenständen (10) wird im nächsten Schritt einschließlich ihrer Umhüllung (30) in einen Sterilisationsraum eingebracht. Erfindungsgemäß können auch zwei oder drei oder eine Vielzahl, zum Beispiel mehr als hundert, oder mehr als zweihundert, oder mehr als dreihundert Gruppen (20) von Gegenständen (10) in den Sterilisationsraum eingebracht werden. Die Gegenstände (10) werden dann dort in geeigneter Weise sterilisiert, Die Sterilisationsbedingungen können dabei auch von der Natur der Gegenstände (10) aber auch von der Art ihrer Anordnung in Gruppen (20), von der Anzahl der Gruppe (20), und von der Art der Umhüllung (30) abhängig sein. Die Gegenstände (10) werden nach Abschluss der Sterilisation, immer noch in der einmal gewählten Gruppenanordnung, dem Sterilisationsraum entnommen. Im nächsten Schritt wird je eine Gruppe (20) von Gegenständen (10) einschließlich (oder zumindest mit Hilfe) ihrer Umhüllung (30) in ein Verpackungsmittel (40) eingebracht. Erfindungsgemäß ist dieses Verpackungsmittel (40) zur vollständigen Umhüllung (30) der Gruppe (20) von Gegenständen (10) geeignet.

In einer alternativen Verfahrensweise wird die vollständige Umhüllung (3) der Gruppe (20) von Gegenständen (10) durch das Verpackungsmittel (40) erst im Zusammenspiel mit der Umhüllung (30) der Gruppe (20) von Gegenständen (10) erreicht. Beispielsweise könnte die Umhüllung (30) eine gut schließende Banderole bilden, die bereits eine großflächige Umhüllung (30) der Gegenstände (10) gestattet, und das Verpackungsmittel (40) ist etwa in Form von Kappen angebracht, welches beispielsweise die Stirnseiten einer Gruppe (20) von Gegenständen (10) aufnimmt.

Bevorzugt ist das Verpackungsmittel (40) auch ohne die Umhüllung (30) zur vollständigen Umhüllung (30) der Gruppe (20) von Gegenständen (10) geeignet. Dies erlaubt es, dass die Umhüllung (30) nach dem Einbringen der Gruppe (20) von Gegenständen (10) in das Verpackungsmittel (40) entfernt wird, Alternativ jedoch kann die Gruppe (20) von Gegenständen (10) einschließlich ihrer Umhüllung (30) vom Verpackungsmittel (40) aufgenommen werden und die Umhüllung (30) verbleibt im Verpackungsmittel (40). An den Schritt des Einbringens der Gruppe (20) von Gegenständen (10) in das Verpackungsmittel (40) schließt sich vorzugsweise das Verschließen des Verpackungsmittels (40) an.

Vorzugsweise ist die Gruppe (20) von Gegenständen (10) die umhüllt wird, identisch mit der Gruppe (20) von Gegenständen (10), die im Sterilisationsraum sterilisiert wird. Es findet also keine Umgruppierung der Gegenstände (10) statt. Weiter ist vorzugsweise diese Gruppe (20) von Gegenständen (10) auch identisch mit der Gruppe (20) von Gegenständen (10), die in das Verpackungsmittel (40) eingebracht wird. Erfindungsgemäß kommt es jedoch in Betracht, dass mehr als eine Gruppe (20) von Gegenständen (10) in das Verpackungsmittel (40) eingebracht wird. Vorzugsweise werden also entweder eine oder zwei oder drei oder vier Gruppen (20) von Gegenständen (10) jeweils in ein Verpackungsmittel (40) eingebracht,

Bevorzugt werden die Gruppen der Erfindung von Gegenständen (10) auf geeigneten Gestellen oder in so genannten Sterilisationskörben eingeordnet und mit deren Hilfe in den Sterilisationsraum eingebracht, vorzugsweise auch mit Hilfe der Gestelle oder Körbe dort sterilisiert und anschließend dem Sterilisationsraum wieder entnommen.

Der Gegenstand der Erfindung ist eine Anlage zur Sterilisation von Gegenständen (10). Diese Anlage führt mit angepassten Vorrichtungen die zuvor beschriebenen Verfahrensschritte vollständig oder jedenfalls zum überwiegenden Teil aus. Bevorzugt arbeiten zumindest die Vorrichtungen gemäß b), c) und f) automatisch, also nicht manuell, sondern maschinell und entsprechend gesteuert.

Die erfindungsgemäße Anlage umfasst zumindest eine automatische Vorrichtung zur Anordnung von Gegenständen (10) in Gruppen (20). Sie umfasst ferner eine automatische Vorrichtung um je eine Gruppe (20) von Gegenständen (10) eine die Gruppe (20) teilweise, aber nicht vollständig bedeckenden Umhüllung (30) versehen. Diese Anlage umfasst ferner Vorrichtungen, um mindestens eine Gruppe (20) von Gegenständen (10) einschließlich der Umhüllung (30) in den Sterilisationsraum einzubringen. Diese Beschickungsvorrichtung kann Sterilisationsgestelle oder Sterilisationskörbe umfassen. Die Beschickungsvorrichtung kann eine vollautomatische Einbringung der Gegenstände (10) in den Sterilisationsraum vorsehen oder die benutzerunterstützte oder vollständig manuelle Einbringung. Die Anlage umfasst ferner eine Sterilisationskammer. Ferner umfasst die erfindungsgemäße Anlage Vorrichtungen zur Entnahme der Gegenstände (10) aus dem Sterilisationsraum. Diese Vorrichtungen können identisch mit denjenigen der Einbringung der Gegenstände (10) in den Sterilisationsraum sein. Die Beschickungs- und die Entnahmevorrichtung können auch teilweise identisch sein, d.h. gemeinsame Teile umfassen, beispielsweise Sterilisationsgestelle und -körbe. Ferner umfasst die erfindungsgemäße Anlage automatische Vorrichtungen, mit denen je eine Gruppe (20) von Gegenständen (10) einschließlich ihrer Umhüllung (30) in ein Verpackungsmittel (40) eingebracht werden können. Vorzugsweise umfasst die erfindungsgemäße Anlage eine Sterilisationskammer, die für die Sterilisierung mit einem Dampf-Luft-Gemisch ausgelegt ist. Es ist weiterhin erfindungsgemäß, dass die Anlage mit all solchen Vorrichtungen ausgestattet werden kann, die es erlauben, das hierin beschriebene Verfahren, einschließlich all seiner bevorzugten Ausgestaltungen durchzuführen.

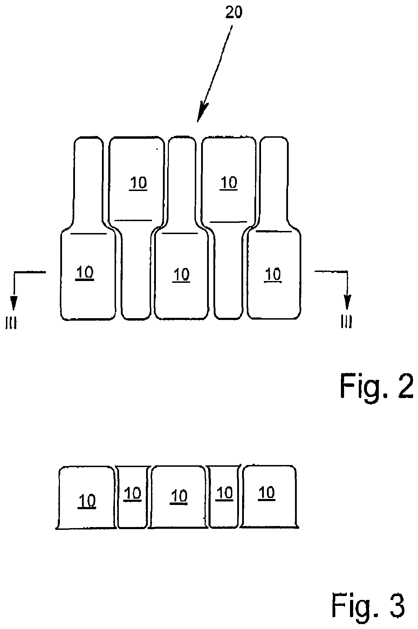

Fig. 2 zeigt in einer Gruppe (20) angeordnete Gegenstände (10), Das vorgestellte Verfahren ist im Prinzip für beliebige Gegenstände (10) geeignet. Es können sowohl gleichartige als auch verschiedenartige Gegenstände (10) gemeinsam sterilisiert werden und in Gruppen (20) angeordnet werden. Vorzugsweise werden jedoch gleichartige Gegenstände (10) in Gruppen (20) angeordnet. Diese Anordnung kann entweder optimiert werden für den Sterilisationsschritt und/oder für die spätere Verpackung. In der Regel ist aber schon für das Einbringen in den Sterilisationsraum eine Raum sparende Anordnung vorteilhaft.
Das Verfahren ist zur Sterilisation von länglichen Gegenständen (10) geeignet. Ein solcher Gegenstand kann zweckmäßig durch seine Hauptachsen, d. h. seine Hauptträgheitsachsen beschrieben werden. Wenn der Gegenstand Symmetrieachsen aufweist, sind diese Symmetrieachsen auch Hauptachsen. Entlang einer Hauptachse wird der Gegenstand die größte Ausdehnung haben. Hierin wird diese Achse als Achse L bezeichnet. Beispielsweise wäre für eine (rotationssymmetrische) Flansche, etwa eine typische Weinflasche, die Achse L die Längsachse, die den Mittelpunkt der Flaschenöffnung mit dem Mittelpunkt des Bodens verbindet. Die länglichen Gegenstände (10) werden so gruppiert, dass ihre Achsen L parallel ausgerichtet sind. Wie die Fig. 2 zeigt, müssen dabei jedoch die Gegenstände (10) nicht alle in die gleiche Richtung ausgelegt werden Insbesondere wenn die Gegenstände (10) Ausbuchtungen und Einbuchtungen aufweisen, ist es häufig vorteilhaft die Gegenstände (10) so auszurichten, dass diese ineinander eingreifen können. In einer bevorzugten Ausführungsfbrm der Erfindung werden längliche Gegenstände (10) mit gegensinnig orientierten Achsen angeordnet. Eine solche Anordnung ist auch in Fig. 2 gezeigt.

Werden die Gegenstände (10) hinreichend beabstandet, führt dies dazu, dass die Gruppe (20) von Gegenständen (10) bei der Sterilisation generell parallel zu den Achsen L von einem Dampf-Luft-Gemisch durchströmt werden kann. Die Strömungskanäle verlaufen allerdings dabei nicht genau parallel zu den Achsen L, sondern sind vielmehr gekrümmt. Um angesichts der Krümmung der Durchströmkanäle die Durchströmung der Gruppe (20) von Gegenständen (10), und damit ihre Sterilisation, nicht weiter zu behindern, ist es vorteilhaft, wenn die Stirnflächen der Gruppe (20) (das sind die Flächen, die senkrecht auf den Achsen L stehen) nicht verdeckt werden. Daher wird erfindungsgemäß die Gruppe (20) der Gegenstände (10) so umhüllt, dass die Umhüllung (30) die Stirnflächen der Gruppe (20) nicht bedeckt. Vorzugsweise beugt dazu die Umhüllung (30) im Wesentlichen den Rand einer oder mehrerer Flächen, die senkrecht zu den Achsen L steht/stehen.

Das Sterilisationsverfahren eignet sich durchaus auch zur Sterilisation von Gegenständen (10), die nicht symmetrisch zur Achse L sind. Dies zeigt auch Fig. 3, welche eine Querschnittsansicht der Gruppe (20) der Gegenstände (10) aus Fig. 2 zeigt. Aus der Fig. 3 ist jedoch auch offenkundig, dass die Gruppe (20) von Gegenständen (10) raumsparend angeordnet ist und Ausbuchungen in Einbuchtungen eingreifen, so dass vollständige oder annähernde Formschlüssigkeit erreicht wird.

Im Sinne der vorliegenden Erfindung wurde erkannt, dass gerade bei einer räumlich optimierten und kompakten Bildung von Gruppen (20) von Gegenständen (10), die gute Durchströmbarkeit wesentlich ist. Ebenfalls kann es wesentlich sein, durch adäquate mechanische Stabilisierung überhaupt eine räumlich optimale Anordnung von Gegenständen (10) zu erzielen und während des Sterilisationsprozesses und Verpackungsprozesses zu erhalten. Das Verfahren hat besondere Vorteile, wenn die zu sterilisierenden Gegenstände (10) länglich sind und die Achse L mehr als doppelt so lang ist wie die anderen Hauptträgheitsachsen.

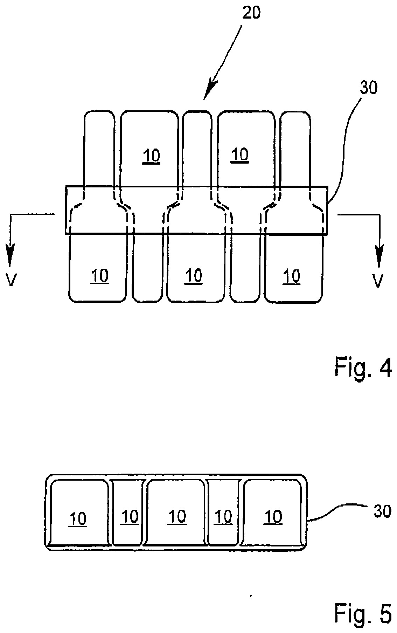

Fig. 4 zeigt in der Draufsicht die vorteilhafte Anbringung einer Umhüllung (30) für die Gruppe (20) von Gegenständen (10), die bereits aus Fig. 2 bekannt ist.

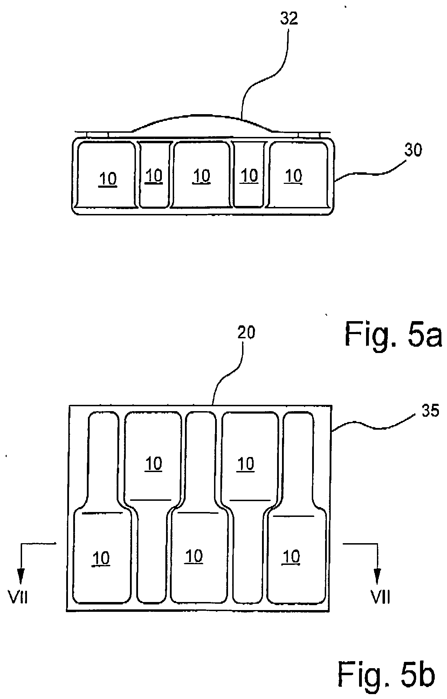

Fig. 5 zeigt eine entsprechende Querschnittsansicht. Wie eng die Umhüllung (30) die Gegenstände (10) umschließen soll, hängt wiederum von der mechanischen Empfindlichkeit der Gegenstände (10) ab, aber auch von strömungstechnischen Erwägungen zum Sterilisationsprozess.

Bevorzugt kann die Umhüllung (30) auch mit einer Transporthilfe oder Greifvorrichtung ausgestattet sein, z. B. einer Griffschlaufe, einem Zapfen oder einer Zapfenaufnahme, einem Magneten oder einem Klettmaterial. Solche Vorrichtungen geben der Umhüllung (30) den Zusatznutzen, dass die Gruppe (20) von Gegenständen (10) leichter transportiert werden und leichter in das Verpackungsmittel (40) eingebracht werden kann. Eine Umhüllung (30) mit Griffschlaufe (32) ist in Fig. 5A gezeigt.

Fig. 5B zeigt ein alternatives Umhüllungskonzept. Die Umhüllung kann ein Stülpboden (35) sein. Zum Einbringen der Gegenstände in das Verpackungsmittel kann der Stülpboden (35) umgestülpt werden.

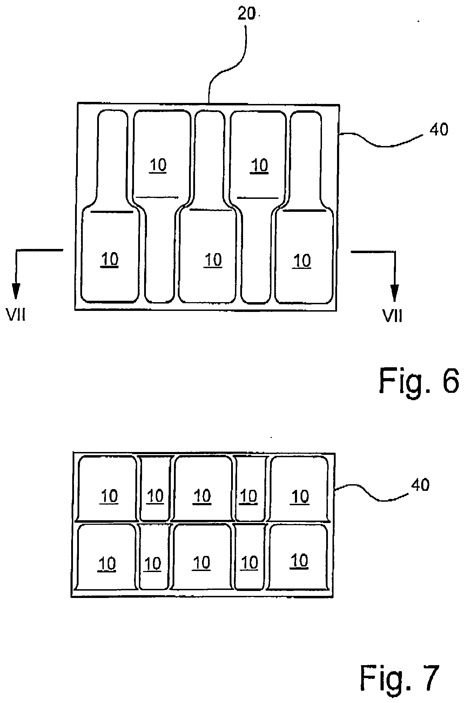

Fig. 6 zeigt in der Draufsicht eine Gruppe (20) von Gegenständen (10), nämlich diejenige die aus Fig. 2 bekannt ist, nachdem sie in ein Verpackungsmittel (40) eingebracht wurde. Wie dargestellt wird die Gruppe (20) von Gegenständen (10) samt der Umhüllung (30) in das Verpackungsmittel (40) eingebracht. Das Verpackungsmittel (40) sollte gerade so viel Platz bieten, dass eine oder mehrere Gruppen (20) von Gegenständen (10) einschließlich der Umhüllung (30) gut aufgenommen werden können. Das so dimensionierte Verpackungsmittel (40) kann die Funktion der mechanischen Stabilisierung der Gruppe (20) von Gegenständen (10) übernehmen. Daher kann die Umhüllung (30) nach dem Einbringen in das Verpackungsmittel (40) entfernt werden,

Alternativ jedoch kann die Umhüllung (30) im Verpackungsmittel (40) verbleiben, d. h., das Verpackungsmittel (40) wird ohne Entfernen der Umhüllung (30) verschlossen.

Fig. 7 zeigt zwei Gruppen (20) von Gegenständen (10), die gemeinsam in ein Verpackungsmittel (40) eingebracht sind. Erfindungsgemäß kann eine einzelne Gruppe (20) von Gegenständen (10) in das Verpackungsmittel (40) eingebracht werden, vorzugsweise werden aber auch zwei oder drei oder vier oder mehrere oder noch mehr Gruppen (20) von Gegenständen (10) in ein Verpackungsmittel (40) eingebracht. Wie es die Fig. 7 zeigt, müssen die Gruppen (20) von Gegenständen (10) dabei nicht in sich identisch angeordnet sein. Erfindungsgemäß können verschieden angeordnete Gruppen (20) gleicher Gegenstände (10) in das Verpackungsmittel (40) eingebracht werden oder auch Gruppen (20) verschiedener Gegenstände (10). Außerdem können Gruppen (20) gleicher Gegenstände (10) verschieden orientiert werden, beispielsweise kann vor dem Einbringen in das Verpackungsmittel (40) eine Gruppe (20) einmal um 90 ° oder 180 ° um eine ihrer Achsen, vorzugsweise Hauptachsen, gedreht werden. Eine solche Anordnung kann zur mechanischen Stabilität der Verpackung einschließlich der Gegenstände (10) beitragen.

An der Fig. 7 wird auch erkennbar, dass die Umhüllung (30) als Originalitätsverschluss gestaltet werden kann. Dazu ist die Umhüllung (30) so auszulegen, dass sie nicht unbemerkt geöffnet werden kann und die Gegenstände (10) nicht unbemerkt aus der Umhüllung (30) entnommen werden können. Ferner ist die Umhüllung (30) vorzugsweise mit dem Verpackungsmittel (40) so zu verbinden, dass eine Entnahme der Umhüllung (30) aus dem Verpackungsmittel (40) sichtbar wird. Vorzugsweise wird die Umhüllung (30) mit einer Kennnummer und einem Haltbarkeitsdatum für die umhüllten Gegenstände (10) (z. B. Ch.-B.-Angabe) versehen. Eine entsprechende Kennung kann auch für das Verpackungsmittel (40) vorgesehen werden.

Die vorliegende Erfindung kann im Zusammenhang mit verschiedenen bekannten Sterilisationsverfahren eingesetzt werden, die zum Sterilisieren von Gruppen (20) von Gegenständen (10) geeignet sind. Besonders vorteilhaft wird es zur Sterilisation mit einem Dampf-Luft-Gemisch eingesetzt und bei Sterilisationstemperaturen im Bereich von 120 bis 140 °C. Besonders vorteilhaft wirkt sich die Erfindung bei Verfahren mit hoher Dampf-Luft-Umwälzgeschwindigkeit und hoher Evakuierungsgeschwindigkeit aus. Das hierin beschriebene und beanspruchte Verfahren zur Sterilisation von Gegenständen kann sehr vorteilhaft bei Verfahren mit Dampf-Luft-Umwälzgeschwindigkeiten von mehr als 3 Metern pro Sekunde oder mehr als 10 Metern pro Sekunde (m/s) eingesetzt werden, besonders Dampf-Luft-Umwälzgeschwindigkeiten im Bereich von 3 m/s bis 300 m/s, vorzugsweise 10 m/s bis 100 m/s, besonders vorzugsweise 20 m/s bis 50 m/s.

Vorteilhaft kann die vorliegende Erfindung bei Dampf-Luft-Sterilisationen eingesetzt werden, bei denen die Sterilisationslcammer von Fugen oder ähnlichen Kältebrücken durchbrochene Wandflächen aufweist. Entsprechende Fugen können Türfugen sein. Es hat sich gezeigt, dass bei solchen Kammern besonders leicht Wasserflecken auftreten.

Im Sinne der vorliegenden Erfindung kann ein Gegenstand durchaus aus vielen Einzelteilen bestehen. Insbesondere kann ein Gegenstand bereits eine individuelle Verpackung aufweisen. Erfindungsgemäß kommen als zu sterilisierende Gegenstände insbesondere Einwegspritzen, auch gefüllte Einwegspritzen, in Frage. Solche Spritzen können individuell zum Beispiel durch eine Blisterverpackung verpackt sein. Solche Blisterverpackungen umfassen eine gewölbte Haube und eine die Haube auf einer Seite verschließende mit ihr verschweißte Schicht. Diese Schicht kann manuell abgezogen werden, um den Inhalt zu entnehmen. Ein Gegenstand im Sinne der Anmeldung ist also beispielsweise, aber auch vorzugsweise, eine gefüllte Einwegspritze in einer Blisterverpackung.

Für die Umhüllung kommt eine Vielzahl an Materialien und Formen in Betracht. Wichtig ist zum einen, dass die Umhüllung eine ausreichende mechanische Stabilität der Gruppe von Gegenständen bewahrt. Insbesondere sollte sie das leichte Handhaben der Gruppe von Gegenständen beim Einbringen in die Verpackung unterstützen. Ferner ist wichtig, dass die Umhüllung die Sterilisation der Gegenstände nur geringfügig beeinträchtigt. Insbesondere sollte die Umhüllung die Durchströmung der Gegenstände mit einem Dampf-Luft-Gemisch nur minimal beeinträchtigen, aber auch die Evakuierung nur minimal beeinträchtigen. Die Umhüllung kann insbesondere als breites oder schmales Band ausgeführt sein (evtl. auch als Litze oder dgl.). Vorzugsweise ist die Umhüllung reißfest, wasserfest und temperaturbeständig bis mindestens 140 °C.

Die Umhüllung selbst sollte im Sterilisationsprozess keinen Schaden leiden und durch denselben Prozess wie die Gegenstände sterilisierbar sein. Die Umhüllung sollte auch so bemessen sein, dass keine Druckspuren an den Gegenständen entstehen. Die Umhüllung kann aus Pappe, Papier oder einem Verbundmaterial hergestellt werden, vorzugsweise aber aus Kunststoff, d. h. polyolefinem Kunststoff, beispielsweise aus Kunststofffolie, insbesondere aus einer Polypropylenfolie. Vorzugsweise ist die Umhüllung aus einem Material, das verschieden von den umhüllten Materialien der Gegenstände ist. Dies vermeidet Wechselwirkungen (z. B. Verkleben oder Verschmelzen) der Materialien während des Sterilisationsverfahrens.

Vorzugsweise weist die Umhüllung auch einen Indikator auf, der die erfolgreiche Sterilisation anzeigt. Wird eine solche Umhüllung verwendet, ist es im Sinne der Erfindung vorzuziehen, wenn diese Umhüllung vor Verschließen des Verpackungsmittels entfernt wird und in weiteren Sterilisationsprozessen wieder verwendet wird.

## Patentansprüche

1. Anlage zur Sterilisation von Gegenständen, vorzugsweise mit Hilfe eines Dampf-Luft-Gemisches, wobei die Gegenstände (10) untereinander gleichartig und länglich sind und je eine Achse L aufweisen, die die längste der Hauptachsen des Gegenstandes (10) ist und mehr als doppelt so lang wie die anderen Hauptachsen des Gegenstandes (10) ist, wobei die Anlage folgende Vorrichtungen umfasst:
a) einen Sterilisationsraum,
b) eine automatische Vorrichtung zur Anordnung der Gegenstände (10) in Gruppen (20),
wobei die Gegenstände (10) in mindestens einer Gruppe (20) so angeordnet werden, dass ihre Hauptachsen L parallel zueinander ausgerichtet sind und
wobei die Gegenstände (10) der Gruppe (20) mit einer Umhüllung versehen werden und wobei die Umhüllung so angebracht wird, dass ein Bündel von Gegenständen entsteht und das Umhüllungsmaterial dem Rand einer Fläche oder den Rändern einer Vielzahl paralleler Flächen folgt, die im Wesentlichen senkrecht zu den Längsachsen der Gegenstände ist oder sind,
c) eine automatische Vorrichtung, um je eine Gruppe (20) von Gegenständen (10) mit einer die Gruppe (20) teilweise, aber nicht vollständig bedeckenden Umhüllung (30) zu versehen,
d) Vorrichtungen, um mindestens eine Gruppe (20) von Gegenständen (10) einschließlich der die Gruppe (20) teilweise, aber nicht vollständig bedeckenden Umhüllung (30) in den Sterilisationsraum einzubringen,
e) Vorrichtungen zur Entnahme der Gegenstände (10) der Gruppe (20) als Bündel einschließlich ihrer Umhüllung (30) aus dem Sterilisationsraum,
f) automatische Vorrichtungen, um je eine Gruppe (20) von Gegenständen (10) einschließlich ihrer Umhüllung (30) in ein Verpackungsmittel (40) einzubringen, das zur vollständigen Umhüllung der Gruppe (20) von Gegenständen (10) geeignet ist.

2. Anlage nach Anspruch 1, **dadurch gekennzeichnet dass** die Vorrichtungen gemäß Merkmal d) einen Sterilisationskorb umfassen.

3. Anlage nach Anspruch 1 oder 2, **dadurch gekennzeichnet, dass** mittels der Anlage nach dem Einbringen der Gruppe (20) von Gegenständen (10) in das Verpackungsmittel (40) das Verpackungsmittel (40) verschlossen wird, wobei vor dem Verschließen des Verpackungsmittels (40) die Umhüllung (30) entfernt wird oder die Umhüllung (30) im Verpackungsmittel (40) verbleibt.

4. Anlage nach einem der vorhergehenden Ansprüche, **dadurch gekennzeichnet, dass** die Umhüllung (30) eine gut schließbare Banderole ist.

5. Anlage nach einem der vorhergehenden Ansprüche, **dadurch gekennzeichnet, dass** die Umhüllung (30) eine Greifvorrichtung aufweist.

6. Anlage nach einem der vorhergehenden Ansprüche, **dadurch gekennzeichnet, dass** die Umhüllung (30) aus polyolefinem Kunststoff besteht.

7. Anlage nach einem der vorhergehenden Ansprüche, **dadurch gekennzeichnet, dass** die Umhüllung (30) einen Indikator aufweist, der eine erfolgreiche Sterilisation anzeigt.

8. Anlage nach einem der vorhergehenden Ansprüche, **dadurch gekennzeichnet, dass** die Anlage auf die Handhabung der Gegenstände (10) in einer individuellen Verpackung, vorzugsweise in einer Blisterverpackung, ausgelegt ist.

9. Anlage nach einem der vorhergehenden Ansprüche, **dadurch gekennzeichnet, dass** die Anlage auf die Handhabung von Einwegspritzen als zu sterilisierende Gegenstände (10) ausgelegt ist.

## Claims

1. An installation for the sterilization of objects, preferably by means of a steam-air mixture, wherein the objects (10) are similar to each other and elongated and each have an axis L, which is the longest of the main axes of the object (10) and more than twice as long as the other main axes of the object (10),
wherein the installation comprises the following devices:
a) a sterilization chamber,
b) an automatic device for the arrangement of the objects (10) in groups (20),
wherein the objects (10) are arranged in at least one group (20) such that their main axes L are aligned parallel to each other and
wherein the objects (10) of the group (20) are provided with a wrapping and wherein the wrapping is applied such that a bundle of objects arises and the wrapping material follows the edge of a surface or the edges of a plurality of parallel surfaces, which is or are substantially perpendicular to the longitudinal axes of the objects,
c) an automatic device, in order to provide each group (20) of objects (10) with a wrapping (30) partially, but not completely covering the group (20),
d) devices, in order to introduce at least one group (20) of objects (10) including the wrapping (30) partially, but not completely covering the group (20) into the sterilization chamber,
e) devices for the removal of the objects (10) of the group (20) as a bundle including their wrapping (30) from the sterilization chamber,
f) automatic devices, in order to introduce each group (20) of objects (10) including their wrapping (30) into a packaging means (40), which is suitable for the complete wrapping of the group (20) of objects (10).

2. An installation according to Claim 1, **characterized in that** the devices according to feature d) comprise a sterilization basket.

3. An installation according to Claim 1 or 2 **characterized in that** by means of the installation after the introduction of the group (20) of objects (10) into the packaging means (40) the packaging means (40) is closed, wherein before the closure of the packaging means (40) the wrapping (30) is removed or the wrapping (30) remains in the packaging means (40).

4. An installation according to any one of the preceding claims, **characterized in that** the wrapping (30) is an easily closable sleeve.

5. An installation according to any one of the preceding claims, **characterized in that** the wrapping (30) has a gripping device.

6. An installation according to any one of the preceding claims, **characterized in that** the wrapping (30) consists of polyolefin plastic.

7. An installation according to any one of the preceding claims, **characterized in that** the wrapping (30) has an indicator, which indicates a successful sterilization.

8. An installation according to any ane of the preceding claims, **characterized in that** the installation is designed for the handling of the objects (10) in an individual package, preferably in a blister package.

9. An installation according to any one of the preceding claims, **characterized in that** the installation is designed for the handling of disposable syringes as objects (10) to be sterilized.

## Revendications

1. Installation de stérilisation d'objets, de préférence à l'aide d'un mélange vapeur-air, les objets (10) et identiques entre eux et allongés et comprenant chacun un axe L, qui est le plus long des axes principaux de l'objet (10) et qui est plus de deux fois plus long que les autres axes principaux de l'objet (10), l'installation comprenant les dispositifs suivante
a) un espace de stérilisation,
b) un dispositif automatique de disposition des objets (10) en groupes (20),
les objets (10) étant disposés dans au moins un groupe (20) de façon à ce que leurs axes principaux L soient orientés parallèlement entre eux et les objets (10) du groupe (20) étant munis d'une enveloppe et l'enveloppe étant posée de façon à ce qu'un ensemble d'objets en résulte et le matériau de l'enveloppe suit le bord d'une surface ou les bords d'une pluralité de surfaces parallèles, qui est ou sont globalement perpendiculaires aux axes longitudinaux des objets,
c) un dispositif automatique afin de munir un groupe (20) d'objets (10) avec une enveloppe (30) recouvrant le groupe (20) partiellement mais pas entièrement,
d) des dispositifs permettant d'insérer dans l'espace de stérilisation au moins un groupe (20) d'objets (10), y compris l'enveloppe (30) recouvrant partiellement mais pas entièrement le groupe (20),
e) des dispositifs pour le retrait des objets (10) du groupe (20) en tant qu'ensemble, y compris leur enveloppe (30), hors de l'espace de stérilisation,
f) des dispositifs automatiques afin d'insérer un groupe (20) d'objets (10), y compris leur enveloppe (30), dans un moyen d'emballage (40) qui est adapté pour l'enveloppement complet du groupe (20) d'objets (10).

2. Installation selon la revendication 1, **caractérisée en ce que** les dispositifs selon la caractéristique d) comprennent une corbeille de stérilisation.

3. Installation selon la revendication 1 ou 2, **caractérisée en ce que**, au moyen de l'installation, après l'insertion du groupe (20) d'objets (10) dans le moyen d'emballage (40), le moyen d'emballage (40) est fermé, moyennant quoi, avant la fermeture du moyen d'emballage (40) l'enveloppe (30) est retirée ou l'enveloppe (30) reste dans le moyen d'emballage (40).

4. Installation selon l'une des revendications précédentes, **caractérisée en ce que** l'enveloppe (30) est une banderole pouvant être bien fermée,

5. Installation selon l'une des revendications précédentes, **caractérisée en ce que** l'enveloppe (30) comprend un dispositif de préhension.

6. Installation selon l'une des revendications précédentes, **caractérisée en ce que** l'enveloppe (30) est constituée d'une matière plastique de type polyoléfine.

7. Installation selon l'une des revendications précédentes, **caractérisée en ce que** l'enveloppe (30) comprend un indicateur qui affiche le succès de la stérilisation.

8. Installation selon l'une des revendications précédentes, **caractérisée en ce que** l'installation est conçus pour le traitement d'objets (10) dans un emballage individuel, de préférence dans un emballage à blister.

9. Installation selon l'une des revendications précédentes, **caractérisée en ce que** l'installation est conçue pour la manipulation de seringues à usage unique en tant qu'objets (10) à stériliser.
